# EUROPEAN PATENT APPLICATION

(11) **EP 4 400 588 A1**
(43) Date of publication of application: **17.07.2024**
(21) Application number: 22867449.5
(22) Date of filing: 09.09.2022
(51) Int. Cl.: C12N 15/54, C12N 1/15, C12N 1/19, C12N 1/21, C12N 5/10, C12N 15/55, C12P 19/32

(54) **RECOMBINANT MICROORGANISM USED FOR PRODUCING CDP-CHOLINE, AND METHOD FOR PRODUCING CDP-CHOLINE USING SAID RECOMBINANT MICROORGANISM**

(30) Priority: 10.09.2021 JP 2021147985
(71) Applicant: Kyowa Hakko Bio Co., Ltd., Tokyo 100-8185 (JP)
(72) Inventor: HORI, Kazumasa, Tokyo 100-8185 (JP); MORITA, Toshihiko, Tokyo 100-8185 (JP); NISHINO, Tsuneyo, Tokyo 100-8185 (JP); UJIHARA, Tetsuro, Tokyo 100-8185 (JP)
(74) Representative: Vossius & Partner Patentanwälte Rechtsanwälte mbB
(86) International application number: PCT/JP2022/033972
(87) International publication number: WO 2023/038128

(57) **Abstract**

Provided are a recombinant microorganism having an ability to produce heterologous CTP:phosphocholine cytidylyltransferase, in which the CTP:phosphocholine cytidylyltransferase is a polypeptide having a CTP:phosphocholine cytidylyltransferase activity and consisting of a specific amino acid sequence, and a method for producing CDP-choline using the recombinant microorganism.

## Description

### TECHNICAL FIELD

The present invention relates to a transformant having an ability to produce a protein having an activity of synthesizing cytidine 5'-diphosphate choline and having excellent substrate specificity, and a method for efficiently producing cytidine 5'-diphosphate choline using the transformant.

### BACKGROUND ART

Cytidine 5'-diphosphate choline (hereinafter referred to as CDP-choline) is a biosynthetic precursor of phosphatidylcholine that is a component constituting phospholipids of higher organisms, and is known to have a nerve protection effect (Non Patent Literature 1).

Even though CDP-choline is useful, it is difficult to obtain a large amount of CDP-choline by extraction from nature or chemical synthesis. CDP-choline is synthesized mainly by an enzyme reaction of an organism using cytidine monophosphate (hereinafter, referred to as CMP) or orotic acid as a starting material (Patent Literatures 1 and 2 and Non Patent Literatures 2 and 3).

When CDP-choline is produced from orotic acid, first, cytidine triphosphate (hereinafter referred to as CTP) is synthesized from orotic acid by a plurality of enzyme reactions. Subsequently, the obtained CTP is condensed by an action of CTP:phosphocholine cytidylyltransferase in the presence of phosphocholine (hereinafter, referred to as P-choline) synthesized from choline by an enzyme reaction, thereby synthesizing CDP-choline (Non Patent Literature 3).

Similarly, when CMP is used as a starting material, CTP is produced from CMP, and is condensed by CTP:phosphocholine cytidylyltransferase in the presence of P-choline, thereby obtaining CDP-choline (Non Patent Literature 3).

From the above, it is understood that CTP:phosphocholine cytidylyltransferase plays an important role in the production of CDP-choline using an enzyme reaction.

CTP:phosphocholine cytidylyltransferases are widely distributed in mammals including humans, eukaryotes such as yeasts, and bacteria (Non Patent Literatures 4, 5, and 6). These CTP:phosphocholine cytidylyltransferases basically have an activity of synthesizing CDP-choline from CTP and P-choline.

However, it is known that, when deoxycytidine triphosphate (hereinafter, referred to as dCTP) is present, the deoxycytidine 5'-diphosphate choline (hereinafter, referred to as dCDP-choline) is produced from dCTP and P-choline by accepting dCTP as a substrate instead of CTP (Non Patent Literature 7).

### CITATION LIST

### PATENT LITERATURE

Patent Literature 1: WO2003/095660
Patent Literature 2: WO2007/023830

### NON PATENT LITERATURE

Non Patent Literature 1: Nutrients (2020) Vol. 12(3), 793, p.1-17
Non Patent Literature 2: Bull. Inst. Chem. Res. Kyoto U. (1976) Vol. 53, p.546-562
Non Patent Literature 3: Appl. Microbiol. Biotechnol. (2017) Vol. 101, p.1409-1417
Non Patent Literature 4: J. Bio. Chem. (1998) Vol. 273, p.14022-14029
Non Patent Literature 5: Eur. J. Biochem. (1987) Vol. 169, p.477-486
Non Patent Literature 6: J. Bio. Chem. (2002) Vol. 277, p.4343-4350
Non Patent Literature 7: Eur. J. Biochem. (1983) Vol. 131, p.223-229

### SUMMARY OF INVENTION

### TECHNICAL PROBLEM

dCTP is an essential compound for life activity as a raw material for DNA synthesis, and therefore, by-production of dCDP-choline in the CDP-choline production using a microorganism cannot be completely prevented, and dCTP is an obstacle to the efficient CDP-choline production. In addition, dCDP-choline has properties extremely similar to those of CDP-choline, and therefore, it is difficult to separate dCDP-choline and CDP-choline.

Therefore, CTP:phosphocholine cytidylyltransferase exhibiting substrate specificity in which reactivity to CTP is higher than reactivity to dCTP is required, but such an enzyme has not been known so far. In addition, although a crystal structure of CTP:phosphocholine cytidylyltransferase of Streptococcus pneumoniae is revealed (Non Patent Literature 6), the reactivity to dCTP cannot be predicted.

An object of the present invention is to provide a recombinant microorganism used for producing CDP-choline and a method for producing CDP-choline using the recombinant microorganism.

### SOLUTION TO PROBLEM

As a result of making studies to solve the above problems, the present inventors have found CTP:phosphocholine cytidylyltransferase which exhibits excellent substrate specificity in which reactivity to CTP is higher than reactivity to dCTP. Further, the present inventors have found that CDP-choline can be efficiently produced by using a recombinant microorganism having an ability to produce heterologous CTP:phosphocholine cytidylyltransferase, and have completed the present invention based on the findings.

That is, the present invention relates to the following.
1. A recombinant microorganism having an ability to produce heterologous CTP:phosphocholine cytidylyltransferase, in which the CTP:phosphocholine cytidylyltransferase is any one polypeptide selected from the following (A1) to (A3) and (B1) to (B3):
   (A1) a polypeptide consisting of the amino acid sequence represented by SEQ ID NO: 10;
   (A2) a polypeptide having a CTP:phosphocholine cytidylyltransferase activity and consisting of an amino acid sequence having an identity of at least 60% or more with the amino acid sequence represented by SEQ ID NO: 10;
   (A3) a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 10;
   (B1) a polypeptide consisting of the amino acid sequence represented by SEQ ID NO: 14;
   (B2) a polypeptide having a CTP:phosphocholine cytidylyltransferase activity and consisting of an amino acid sequence having an identity of at least 60% or more with the amino acid sequence represented by SEQ ID NO: 14; and
   (B3) a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 14.
2. The recombinant microorganism according to the above 1, in which any one DNA selected from the following (a1) to (a3) and (b1) to (b3) is introduced in an expressible manner:
   (a1) a DNA encoding the amino acid sequence represented by SEQ ID NO: 10;
   (a2) a DNA that encodes a polypeptide having a CTP:phosphocholine cytidylyltransferase activity and consisting of an amino acid sequence having an identity of at least 60% or more with the amino acid sequence represented by SEQ ID NO: 10;
   (a3) a DNA that encodes a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 10;
   (b1) a DNA encoding the amino acid sequence represented by SEQ ID NO: 14;
   (b2) a DNA that encodes a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 14; and
   (b3) a DNA that consists of a DNA having an identity of at least 60% or more with the DNA encoding the amino acid sequence represented by SEQ ID NO: 14, and encodes a polypeptide having a CTP:phosphocholine cytidylyltransferase activity.
3. The recombinant microorganism according to the above 1 or 2, which is Escherichia coli.
4. The recombinant microorganism according to any one of the above 1 to 3, in which the CTP:phosphocholine cytidylyltransferase has reactivity to dCTP of 0.75 or less when reactivity to CTP is defined as 1.
5. A method for producing CDP-choline, the method including producing CDP-choline using the recombinant microorganism according to any one of the above 1 to 4.

### ADVANTAGEOUS EFFECTS OF INVENTION

A recombinant microorganism of the present invention has an ability to produce heterologous CTP:phosphocholine cytidylyltransferase which exhibit excellent substrate specificity in which reactivity to CTP is higher than reactivity to dCTP. The by-production of dCDP-choline can be prevented and CDP-choline can be efficiently produced by using the recombinant microorganism of the present invention.

### BRIEF DESCRIPTION OF DRAWINGS

[Figure] Figure is a diagram showing reactivity of SplicC, LslicC, or AhlicC to CTP or dCTP. The vertical axis represents a ratio of an amount of dCDP-choline to an amount of CDP-choline produced using CTP or dCTP and P-choline as substrates. SplicC, LslicC, and AhlicC represent CTP:phosphocholine cytidylyltransferases derived from Streptococcus pneumoniae, Lactobacillus salivarius, and Anaerococcus hydrogenalis, respectively.

### DESCRIPTION OF EMBODIMENTS

### 1. Recombinant Microorganism and Method for Forming Same

The recombinant microorganism of the present invention is a recombinant microorganism having an ability to produce heterologous CTP:phosphocholine cytidylyltransferase. In the present invention, the CTP:phosphocholine cytidylyltransferase is any one polypeptide selected from the following (A1) to (A3) and (B1) to (B3).
(A1) a polypeptide consisting of the amino acid sequence represented by SEQ ID NO: 10;
(A2) a polypeptide having a CTP:phosphocholine cytidylyltransferase activity and consisting of an amino acid sequence having an identity of at least 60% or more with the amino acid sequence represented by SEQ ID NO: 10;
(A3) a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 10;
(B1) a polypeptide consisting of the amino acid sequence represented by SEQ ID NO: 14;
(B2) a polypeptide having a CTP:phosphocholine cytidylyltransferase activity and consisting of an amino acid sequence having an identity of at least 60% or more with the amino acid sequence represented by SEQ ID NO: 14; and
(B3) a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 14.

Examples of the polypeptide described in the above (A2) or (B2) include a polypeptide having a CTP:phosphocholine cytidylyltransferase activity and consisting of an amino acid sequence having an identity of at least 60% or more, preferably 70% or more, 80% or more, 90% or more, 95% or more in this order, more preferably 98% or more, and most preferably 99% or more, with the amino acid sequence represented by SEQ ID NO: 10 or 14.

Examples of the polypeptide described in the above (A3) or (B3) include a polypeptide having a CTP:phosphocholine cytidylyltransferase activity and consisting of an amino acid sequence in which 1 to 20, preferably 1 to 10, further preferably 1 to 8, and most preferably 1 to 5 amino acids are deleted, substituted, inserted, or added in the amino acid sequence represented by SEQ ID NO: 10 or 14.

The identity of the amino acid sequence and the nucleotide sequence can be determined by using Lipman-Pearson method [Science, 227 (4693), 1435-41 (1985)], Algorithm BLAST by Karlin and Altschul [Pro. Natl. Acad. Sci. USA, 90, 5873 (1993)] or FASTA [Methods Enzymol., 183, 63 (1990)]. Based on the algorithm BLAST, a program called BLASTN or BLASTX has been developed [J. Mol. Biol., 215, 403 (1990)]. When the nucleotide sequence is analyzed by BLASTN based on BLAST, the parameters are, for example, Score = 100 and wordlength = 12. When the amino acid sequence is analyzed by BLASTX based on BLAST, the parameters are, for example, score = 50 and wordlength = 3. When BLAST program and Gapped BLAST programs are used, default parameters for each program are used. A specific method of the analysis method is known.

The amino acid sequence in which an amino acid is deleted, substituted, inserted, or added refers to an amino acid sequence obtained by artificially deleting or substituting an amino acid residue from an original amino acid sequence or artificially inserting or adding an amino acid residue into the original amino acid sequence.

The amino acid to be deleted, substituted, inserted, or added may be of a natural type or a non-natural type. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

The CTP:phosphocholine cytidylyltransferase activity refers to an activity of synthesizing CDP-choline from CTP and P-choline.

It can be confirmed, for example, by the following method that the polypeptide has a CTP:phosphocholine cytidylyltransferase activity. First, a recombinant DNA containing a DNA encoding the polypeptide is prepared by the method described later. Next, a microorganism obtained by transforming a microorganism whose CTP:phosphocholine cytidylyltransferase activity cannot be confirmed, for example, an Escherichia coli BL21 strain, with the recombinant DNA is cultured, and a cell extract containing the polypeptide is prepared from the obtained culture. Subsequently, the cell extract is brought into contact with CTP and P-choline, which are substrates, to produce CDP-choline. Finally, it can be confirmed that the target polypeptide has a CTP:phosphocholine cytidylyltransferase activity by detecting CDP-choline in the reaction solution by a general analysis method using high performance chromatography, gas chromatography, or the like.

The "recombinant microorganism having an ability to produce phosphocholine cytidylyltransferase" can be obtained by introducing a recombinant DNA having a DNA encoding the CTP:phosphocholine cytidylyltransferase into a parent strain in an expressible manner.

The parent strain refers to an original strain to be subjected to genetic modification, transformation, and the like. A strain to be subjected to transformation by introduction of a DNA is referred to as a host strain.

The parent strain is preferably a prokaryote or a yeast strain, and more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like. Among them, a prokaryote belonging to the genus Escherichia (Escherichia coli) is preferred.

Specific examples of the parent strain include prokaryotes such as Escherichia coli BL21 codon plus, Escherichia coli XL1-Blue, Escherichia coli XL2-Blue (all manufactured by Agilent Technologies, Inc.), Escherichia coli BL21 (DE3) pLysS (manufactured by Merck Millipore Inc.), Escherichia coli BL21, Escherichia coli DH5α, Escherichia coli HST08 Premium, Escherichia coli HST02, Escherichia coli HST04 dam-/dcm-, Escherichia coli JM109, Escherichia coli HB101, Escherichia coli CJ236, Escherichia coli BMH71-18 mutS, Escherichia coli MV1184, Escherichia coli TH2 (all manufactured by Takara Bio Inc.), Escherichia coli W (ATCC9637), Escherichia coli JM101, Escherichia coli W3110, Escherichia coli MG1655, Escherichia coli DH1, Escherichia coli MC1000, Escherichia coli W1485, Escherichia coli MP347, Escherichia coli NM522, Serratia ficaria, Serratia fonticola, Serratia liquefaciens, Serratia marcescens, Bacillus subtilis, Bacillus amyloliquefaciens, Brevibacterium immariophilum ATCC 14068, Brevibacterium saccharolyticum ATCC14066, Corynebacterium ammoniagenes, Corynebacterium glutamicum ATCC 13032, Corynebacterium glutamicum ATCC 14067, Corynebacterium glutamicum ATCC13869, Corynebacterium acetoacidophilum ATCC13870, Microbacterium ammoniaphilumATCC15354, or Pseudomonas sp.D-0110, and yeast strains such as Saccharomyces cerevisiae, Schizosaccharomyces pombe, Kluyveromyces lactis, Trichosporon pullulans, Schwanniomyces alluvius, Pichia pastoris, or Candida utilis.

In addition, as the parent strain, a breeding strain to which an ability to generate CTP and/or P-choline, which are substrates of the CTP:phosphocholine cytidylyltransferase, is artificially imparted or a breeding strain whose ability to produce CTP and/or P-choline is enhanced can also be preferably used.

Examples of a method for artificially imparting or enhancing the ability to produce CTP and/or P-choline in a microorganism used as a parent strain include the following (1) to (5), and the above-described known methods may be used alone or in combination.
(1) A method of relaxing or releasing at least one mechanism for controlling a biosynthetic pathway of CTP and/or P-choline
(2) A method of enhancing expression of at least one enzyme associated with the biosynthetic pathway of CTP and/or P-choline
(3) A method of increasing the number of copies of at least one enzyme gene associated with the biosynthetic pathway of CTP and/or P-choline
(4) A method of weakening or blocking at least one metabolic pathway branching off from the biosynthetic pathway of CTP and/or P-choline to a metabolic product other than a target substance
(5) A method of selecting a cell line having higher resistance to CTP and/or P-choline analogs than a wild strain

Specific examples of the method for artificially imparting or enhancing the ability to produce CTP and P-choline include a known method such as a method using various genetic manipulations (Patent Literature 1, Patent Literature 2, and Non Patent Literature 3).

It can be confirmed that the microorganism is a microorganism capable of producing CTP and P-choline by culturing a breeding strain and a parent strain in respective culture media and comparing a production amount of CTP and a production amount of P-choline. It can also be confirmed by transforming the microorganism with a recombinant DNA containing a DNA encoding a polypeptide consisting of the amino acid sequence represented by SEQ ID NO: 10 or 14, culturing the transformed microorganism in a culture medium, and detecting CDP-choline accumulated in the culture by HPLC described later.

When a microorganism used as the parent strain has an ability to produce a polypeptide having a CTP:phosphocholine cytidylyltransferase activity other than the above (A1) to (A3) and (B1) to (B3), CDP-choline can be efficiently produced while preventing the by-production of dCDP-choline by using, as the parent strain, a strain in which a function of the polypeptide is degraded or deleted. The method for degrading or deleting the function of the polypeptide is not particularly limited as long as the function of the polypeptide is reduced or completely stopped. For example, a known method such as a method of removing all or a part of a DNA encoding the polypeptide can be used as appropriate.

Examples of a DNA encoding the CTP:phosphocholine cytidylyltransferase include any one selected from the following (a1) to (a3), (b1) to (b3), and (c1) to (c3).
(a1) a DNA encoding the amino acid sequence represented by SEQ ID NO: 10;
(a2) a DNA that encodes a polypeptide having a CTP:phosphocholine cytidylyltransferase activity and consisting of an amino acid sequence having an identity of at least 60% or more with the amino acid sequence represented by SEQ ID NO: 10;
(a3) a DNA that encodes a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 10;
(b1) a DNA encoding the amino acid sequence represented by SEQ ID NO: 14;
(b2) a DNA that encodes a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 14; and
(b3) A DNA that consists of a DNA having an identity of at least 60% or more with the DNA encoding the amino acid sequence represented by SEQ ID NO: 14, and encodes a polypeptide having a CTP:phosphocholine cytidylyltransferase activity
(c1) A DNA having the nucleotide sequence represented by SEQ ID NO: 7 or 11
(c2) A DNA that hybridizes with a DNA consisting of a nucleotide sequence complementary to the nucleotide sequence represented by SEQ ID NO: 7 or 11 under stringent conditions, and encodes a polypeptide having a CTP:phosphocholine cytidylyltransferase activity
(c3) A DNA consisting of a nucleotide sequence having an identity of 95% or more, preferably 97% or more, more preferably 98% or more, and most preferably 99% or more with the nucleotide sequence represented by SEQ ID NO: 7 or 11, and encoding a DNA having a CTP:phosphocholine cytidylyltransferase activity

The term "hybridizing" means that a DNA hybridizes to a DNA having a specific nucleotide sequence or a part of the DNA. Accordingly, the DNA having a specific nucleotide sequence or a part thereof is a DNA that can be used as a probe for Northern or Southern blot analysis, or a DNA that can be used as an oligonucleotide primer for PCR analysis.

Examples of the DNA used as a probe include DNAs having at least 100 bases or more, preferably 200 bases or more, and more preferably 500 bases or more. Examples of the DNA used as a primer include DNAs having at least 10 bases or more, and preferably 15 bases or more.

A method for a DNA hybridization experiment is well known, and for example, Molecular Cloning, 4th Edition (Cold Spring Harbor Laboratory Press (2012)), Methods for General and Molecular Bacteriology (ASM Press (1994)), Immunology methods manual (Academic press (1997)), and many other standard textbooks can be followed to determine hybridization conditions and perform experiments.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mM sodium chloride, 75 mM sodium citrate), 50 mM sodium phosphate (pH 7.6), a 5× Denhardt's solution, 10% dextran sulfate, and a 20 µg/l of denatured salmon sperm DNA, and then the filter is washed in a 0.2×SSC solution at, for example, about 65°C.

The above various conditions can also be set by adding or changing a blocking reagent used to reduce background in a hybridization experiment. The addition of the above blocking reagent may be accompanied by a change in hybridization conditions in order to meet the conditions.

Examples of the DNA capable of hybridizing under the above stringent conditions include DNAs having an identity of at least 95% or more, preferably 97% or more, more preferably 98% or more, and most preferably 99% or more with the DNA consisting of the nucleotide sequence represented by SEQ ID NO: 7 or 11 when calculated based on the parameters or the like using BLAST, FASTA, or the like.

The DNA described in the above (a1) or (c1) among DNAs encoding CTP:phosphocholine cytidylyltransferase can be obtained by, for example, Southern hybridization for a chromosomal DNA library of a microorganism using a probe DNA that can be designed based on the amino acid sequence represented by SEQ ID NO: 10 or the nucleotide sequence represented by SEQ ID NO: 7, or PCR [PCR protocols, Academic Press (1990)] using, as a template, a chromosomal DNA of a microorganism using a primer DNA that can be designed based on the nucleotide sequence. The origin of the chromosomal DNA of the microorganism used in the above operation is not particularly limited, and examples thereof include lactic acid bacteria of the genus Lactobacillus, and bacteria of the genus Anaerococcus, the genus Streptococcus, the genus Fusobacterium, the genus Bacillus, the genus Paenibacillus, the genus Granulicatella, and the genus Clostridium. Among them, lactic acid bacteria of the genus Lactobacillus and bacteria of the genus Anaerococcus are preferred, and Lactobacillus salivarius is more preferred. Lactobacillus salivarius ATCC 11741 strain is available from the American Type Culture Collection (ATCC).

The DNA described in (b1) or (c1) among the DNAs encoding CTP:phosphocholine cytidylyltransferase can be obtained by, for example, Southern hybridization for a chromosomal DNA library of a microorganism using a probe DNA that can be designed based on the amino acid sequence represented by SEQ ID NO: 14 or the nucleotide sequence represented by SEQ ID NO: 11, or PCR using, as a template, a chromosomal DNA of a microorganism using a primer DNA that can be designed based on the nucleotide sequence. The origin of the chromosomal DNA of the microorganism used in the above operation is not particularly limited, and examples thereof include lactic acid bacteria of the genus Lactobacillus, and bacteria of the genus Anaerococcus, the genus Streptococcus, the genus Fusobacterium, the genus Bacillus, the genus Paenibacillus, the genus Granulicatella, and the genus Clostridium. Among them, lactic acid bacteria of the genus Lactobacillus and bacteria of the genus Anaerococcus are preferred, and Anaerococcus hydrogenalis is more preferred. Anaerococcus hydrogenalis ATCC49630 strain is available from the American Type Culture Collection (ATCC).

The DNA described in the above (a2), (b2), or (c2) among the DNAs encoding CTP:phosphocholine cytidylyltransferase can be obtained by a method using Southern hybridization or PCR similar to the above method for obtaining the DNA by, for example, searching various protein sequence databases for an amino acid sequence having an identity of 60% or more, preferably 70% or more, 80% or more, 90% or more, 95% or more in this order, more preferably 98% or more, and most preferably 99% or more with the amino acid sequence represented by SEQ ID NO: 10 or 14, or searching various gene sequence databases for a nucleotide sequence having an identity of 95% or more, preferably 97% or more, more preferably 98% or more, and most preferably 99% or more with the nucleotide sequence represented by SEQ ID NO: 7 or 11, and using a probe DNA or a primer DNA that can be designed based on the amino acid sequence or the nucleotide sequence obtained by the search, and a microorganism having the DNA.

The DNA described in the above (a3), (b3), or (c3) among DNAs encoding CTP:phosphocholine cytidylyltransferase can be obtained by, for example, subjecting a DNA consisting of a nucleotide sequence encoding the amino acid sequence represented by SEQ ID NO: 10 or 14 or the nucleotide sequence represented by SEQ ID NO: 7 or 11 to error-prone PCR or the like as a template.

The DNA described in the above (a3), (b3), or (c3) can also be obtained by PCR [Gene, 77, 51 (1989)] using a set of PCR primers having, at the 5' end thereof, a nucleotide sequence designed to insert a target mutation (deletion, substitution, insertion, or addition). That is, first, PCR is performed using the DNA as a template with a sense primer corresponding to the 5' end of the DNA consisting of the nucleotide sequence encoding the amino acid sequence represented by SEQ ID NO: 10 or 14 or the nucleotide sequence represented by SEQ ID NO: 7 or 11 and an antisense primer having a sequence complementary to the mutation sequence at the 5' end and corresponding to a sequence immediately before (5' side) a mutation introduction site, to amplify a fragment A from the 5' end of the DNA to the mutation introduction site (the mutation is introduced to the 3' side). Next, PCR is performed using the DNA as a template with a sense primer having a mutation sequence at the 5' end and corresponding to a sequence immediately after the mutation introduction site (3' side) and an antisense primer corresponding to the 3' end of the DNA, to amplify a fragment B from the mutation introduction site to the 3' end of the DNA having the mutation introduced at the 5' end. When these amplified fragments are purified, mixed, and subjected to PCR without adding a template or a primer, a sense strand of the amplified fragment A and an antisense strand of the amplified fragment B hybridize because they have the same mutation introduction site, and the PCR reaction proceeds with the hybridized fragment as a primer and as a template to amplify the DNA into which the mutation is introduced.

A nucleotide sequence of the DNA can be determined by using the obtained DNA described in the above (a1) to (a3), (b1) to (b3), and (c1) to (c3) as it is or cleaving the DNA with an appropriate restriction enzyme or the like, incorporating the DNA into a vector by an ordinary method, introducing the obtained recombinant DNA into a host cell, and then analyzing the DNA using a nucleotide sequence analysis method generally used, such as a dideoxy method [Proc. Natl. Acad. Sci., USA, 74, 5463 (1977)] or a nucleotide sequence analyzer such as an Applied Biosystems 3500 Genetic Analyzer and an Applied Biosystems 3730 DNA Analyzer (both manufactured by Thermo Fisher Scientific K.K.).

Examples of the host cell used when determining the nucleotide sequence of the DNA include Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, Escherichia coli TH2 (all manufactured by Takara Bio Inc.), Escherichia coli XL1-Blue, Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

Examples of the above vector include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT [Nucleic Acids Res., 18, 6069 (1990)].

As a method for introducing a recombinant DNA obtained by incorporating the DNA of the present invention into a host cell, any method can be used as long as it is a method for introducing a DNA into a host cell. Examples thereof include a method using calcium ions (Proc. Natl. Acad. Sci., USA, 69, 2110, 1972), a protoplast method (JPS63-248394A), and an electroporation method (Nucleic Acids Res., 16, 6127, 1988).

When the obtained DNA is partial length as a result of determining the nucleotide sequence, the full-length DNA can be obtained by a Southern hybridization method or the like for a chromosomal DNA library using the partial-length DNA as a probe.

Furthermore, a target DNA can also be prepared by chemical synthesis using an NTS M series DNA synthesizer or the like manufactured by Nihon Techno Service Co., Ltd. based on the determined DNA nucleotide sequence or the nucleotide sequence represented by SEQ ID NO: 7 or 11.

The recombinant DNA having the DNAs described in the above (a1) to (a3), (b1) to (b3), and (c1) to (c3) is, for example, a DNA in which the DNA is autonomously replicable in a parent strain, and in which DNA described in any one or more of (a1) to (a3), (b1) to (b3), and (c1) to (c3) is incorporated into an expression vector containing a promoter at a position where the DNA described in any one or more of (a1) to (a3), (b1) to (b3), and (c1) to (c3) can be transcribed.

The recombinant DNA that can be incorporated into a chromosome in a parent strain, and has the DNA described in any one or more of (a1) to (a3), (b1) to (b3), and (c1) to (c3) is also the recombinant DNA having the DNAs described in the above (a1) to (a3), (b1) to (b3), and (c1) to (c3). When the recombinant DNA is a DNA that can be incorporated into the chromosomal DNA of the parent strain, a promoter may not be contained.

When prokaryotes such as bacteria are used as parent strains, the recombinant DNA that is autonomously replicable in the parent strains is preferably a recombinant DNA containing a promoter, a ribosome binding sequence, the DNA described in any one or more of (a1) to (a3), (b1) to (b3), and (c1) to (c3), and a transcription termination sequence. A gene controlling the promoter may be contained. It is preferable to use a recombinant DNA in which a distance between a Shine-Dalgarno sequence, which is a ribosome binding sequence, and a start codon is adjusted to an appropriate distance (for example, 6 to 18 bases).

In the recombinant DNA that is autonomously replicable in the parent strains, a transcription termination sequence is not necessarily required for expression of the DNA, but it is preferable to place the transcription termination sequence immediately after a structural gene.

The expression vector is not particularly limited as long as it is an appropriate nucleic acid molecule for introducing the target DNA into a host, and causing the target DNA to be amplified and expressed. For example, not only plasmids, but also, for example, artificial chromosomes, vectors using transposons, and cosmids may be used.

When a microorganism belonging to the genus Escherichia is used as the parent strain, examples of the expression vector include pColdI, pSTV28, pSTV29, pUC118 (all manufactured by Takara Bio Inc.), pMW119 (manufactured by Nippon Gene Co., ltd.), pET21a, pCOLADuet-1, pCDFDuet-1, pCDF-1b, pRSF-1b (all manufactured by Merck Millipore Inc.), pMAL-c5x (manufactured by New England Biolabs), pGEX-4T-1, pTrc99A (both manufactured by GE Healthcare Bioscience), pTrcHis, pSE280 (both manufactured by Thermo Fisher Scientific K.K.), pGEMEX-1 (manufactured by Promega Corporation), pQE-30, pQE80L (both manufactured by Qiagen), pET-3, pBluescriptII SK(+), pBluescriptII KS(-) (all manufactured by Agilent Technologies, Inc.), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pTrS30 [prepared from Escherichia coli JM109/pTrS30(FERM BP-5407)], pTrS32 [prepared from Escherichia coli JM109/pTrS32(FERM BP-5408)], pTK31 [APPLIED AND ENVIRONMENTAL MICROBIOLOGY, 2007, Vol.73, No.20, p6378-6385], pPAC31 (WO98/12343), pUC19 [Gene, 33, 103 (1985)], and pPA1 (JPS63-233798A).

The promoter in the case of using the above expression vector may be any promoter as long as it functions in cells of microorganisms belonging to the genus Escherichia, and examples thereof include a promoter of a gene associated with amino acid biosynthesis, such as a trp promoter or an ilv promoter, and a promoter derived from, for example, an Escherichia coli or a phage, such as a uspA promoter, a lac promoter, a PL promoter, a PR promoter, or a PSE promoter. A promoter that is artificially modified in design, such as a promoter with two trp promoters in series, a tac promoter, a trc promoter, a lacT7 promoter, or a letI promoter can also be used.

When a coryneform bacterium is used as the parent strain, examples of the expression vector include pCG1 (JPS57-134500A), pCG2 (JPS58-35197A), pCG4 (JPS57-183799A), pCG11 (JPS57-134500A), pCG 116, pCE54, and pCB101 (all JPS58-105999A), pCE51, pCE52, and pCE53 [all Molecular and General Genetics, 196, 175, (1984)].

The promoter in the case of using the above expression vector may be any promoter as long as it functions in cells of the coryneform bacterium, and examples thereof include a P54-6 promoter [Appl. Microbiol. Biotechnol., 53, p674-679, (2000)].

When a yeast strain is used as the parent strain, examples of the expression vector include YEp13 (ATCC37115), YEp24 (ATCC37051), YCp50 (ATCC37419), pHS19, and pHS15.

The promoter in the case of using the above expression vector may be any promoter as long as it functions in cells of the yeast strain, and examples thereof include a PHOS promoter, a PGK promoter, a GAP promoter, an ADH promoter, a gall promoter, a gal10 promoter, a heat shock polypeptide promoter, an MFα1 promoter, and a CUP1 promoter.

The recombinant DNA of the present invention can be prepared by, for example, subjecting the DNA fragment prepared by the above-described method to a restriction enzyme treatment or the like and inserting the DNA fragment into downstream of the promoter of the appropriate expression vector.

Here, the expression level of the protein encoded by the DNA can also be improved by substituting a base such that the nucleotide sequence of the DNA is an optimal codon for expression in a host. Information on the frequency of codon use in the parent strain used in the production method of the present invention can be obtained through a public database.

Examples of the method for introducing the recombinant DNA having the DNA encoding CTP:phosphocholine cytidylyltransferase as an autonomously replicatable plasmid in a host cell include the above-described method using calcium ions, protoplast method, and electroporation method, a spheroplast method [Proc. Natl. Acad. Sci., USA, 81, 4889 (1984)], and a lithium acetate method [J. Bacteriol., 153, 163 (1983)].

When a recombinant DNA having a DNA encoding CTP:phosphocholine cytidylyltransferase is inserted into a genome of the parent strain, for example, a method according to homologous recombination may be used. That is, a DNA to which a part of the chromosomal region that causes the introduction of the target DNA is bound is incorporated into a microorganism cell, and homologous recombination in a part of the chromosomal region is caused, so that the DNA can be incorporated into the genome. Examples of the method using homologous recombination frequently used in Escherichia coli include a method of introducing a recombinant DNA using a homologous recombination system of a lambda phage [Proc. Natl. Acad. Sci. USA, 97, 6641-6645 (2000)]. Here, the chromosomal region that causes introduction is not particularly limited, and is preferably an unessential genetic region or a non-genetic region upstream of the unessential genetic region. As a method of incorporating the DNA into a microorganism cell, any method can be used as long as it is a method of introducing the DNA into a host cell. Examples thereof include the above-described method using calcium ions, protoplast method, and electroporation method.

It can be confirmed that the microorganism is a microorganism obtained by introducing, into a parent strain, the recombinant DNA having a DNA encoding CTP:phosphocholine cytidylyltransferase in an expressible manner, for example, by comparing the transcription level of the DNA of the microorganism with that of the parent strain by means of Northern blotting or by comparing the production level of the protein of the microorganism with that of the parent strain by means of Western blotting.

It can be confirmed by, for example, the following method that the microorganism produced by the above method is a recombinant microorganism having the ability to produce CTP:phosphocholine cytidylyltransferase. First, the parent strain and the formed microorganism are cultured in respective culture media, and a cell extract containing CTP:phosphocholine cytidylyltransferase is prepared from the obtained culture. Subsequently, the cell extract is brought into contact with CTP and P-choline, which are substrates, to produce CDP-choline. Finally, by detecting CDP-choline in the reaction solution by a general analysis method using high performance chromatography, gas chromatography, or the like, it can be confirmed that the formed microorganism is a recombinant microorganism having an ability to produce CTP:phosphocholine cytidylyltransferase.

Specific examples of the recombinant microorganisms having the ability to produce heterologous CTP:phosphocholine cytidylyltransferase include BL21/pET21a-LslicC and BL21/pET21a-AhlicC described later in Examples.

The CTP:phosphocholine cytidylyltransferase in the present invention preferably has high substrate specificity and reactivity to CTP higher than reactivity to dCTP. Specifically, for example, when the reactivity to CTP is defined as 1, the reactivity to dCTP is preferably 0.75 or less, more preferably 0.60 or less, 0.50 or less, 0.40 or less in this order, further preferably 0.30 or less, particularly preferably 0.20 or less, and most preferably 0.15 or less. The reactivity to dCTP when the reactivity to CTP is defined as 1 can be calculated from, for example, [an amount of dCDP-choline produced when CTP:phosphocholine cytidylyltransferase, P-choline, and dCTP coexist (mM)]/[an amount of CDP-choline produced when CTP:phosphocholine cytidylyltransferase, P-choline, and CTP coexist (mM)].

As described above, CDP-choline can be efficiently produced while preventing the by-production of dCDP-choline by using a recombinant microorganism having the ability to produce CTP:phosphocholine cytidylyltransferase which exhibits substrate specificity in which the reactivity to CTP is higher than the reactivity to dCTP.

### 2. Method for Producing CDP-choline

The method for producing CDP-choline of the present invention includes producing CDP-choline using the recombinant microorganism having an ability to produce the above heterologous CTP:phosphocholine cytidylyltransferase.

As a method for producing CDP-choline, a fermentation method or a method using a biocatalyst has been commonly known (for example, JP3369236B; Y. Liu et al. Appl. Microbiol. Biotechnol., 101, 1409, 2017.). According to the method for producing CDP-choline of the present invention, by-production of dCDP-choline can be more effectively prevented and CDP-choline can be more efficiently produced than in the method according to the related art by using a recombinant microorganism having the ability to produce heterologous CTP:phosphocholine cytidylyltransferase with high reactivity to CTP as compared with reactivity to dCTP. Specifically, for example, in the method for producing CDP-choline described in JP3369236B and Y. Liu et al. Appl. Microbiol. Biotechnol., 101, 1409, 2017., the effect of the present invention can be obtained by using the CTP:phosphocholine cytidylyltransferase in the present invention instead of choline phosphate cytidylyltransferase (CCT).

Examples of the method for producing CDP-choline of the present invention include (I) a method for producing CDP-choline by the fermentation method, and (II) a method for producing CDP-choline using a culture of a recombinant microorganism and a treated product of the culture. Hereinafter, each production method will be described.

### (I) Method for Producing CDP-choline by Fermentation Method

The method for producing CDP-choline by the fermentation method include a method for producing CDP-choline characterized in that recombinant microorganisms having an ability to produce heterologous CTP:phosphocholine cytidylyltransferase are cultured in a culture medium to produce CDP-choline in the culture. The production method may include, for example, producing CDP-choline in the culture, then accumulating CDP-choline, and collecting CDP-choline from the culture.

As the recombinant microorganisms to be used in the method for producing CDP-choline by the fermentation method, the recombinant microorganisms of the present invention in the above 1 are preferably used. In addition, microorganisms having an ability to produce CTP and P-choline that are substrates of the CTP:phosphocholine cytidylyltransferases and/or microorganisms with artificially weakened degrading activity of CTP, P-choline, or CDP-choline are preferably used.

The microorganism can be cultured according to an ordinary method. As a culture medium for culturing the microorganism, either a natural culture medium or a synthetic culture medium may be used as long as the culture medium contains a substrate of the CTP:phosphocholine cytidylyltransferase or a starting material of the substrate, a carbon source that can be assimilated by the microorganism, a nitrogen source, an inorganic salt, and the like, and the microorganism can be efficiently cultured on the culture medium.

Examples of the substrate of the CTP:phosphocholine cytidylyltransferase include CTP and P-choline. Examples of the starting material of the substrate include orotic acid, CMP, choline chloride, and glucose. The carbon source is not particularly limited as long as it can be assimilated by the microorganisms, and examples thereof include: carbohydrates such as glucose, fructose, sucrose, molasses containing the same, starch, and starch hydrolysates; organic acids such as acetic acid and propionic acid; and alcohols such as ethanol and propanol.

Examples of the nitrogen source include ammonium salts of inorganic acids or organic acids, such as ammonia, ammonium chloride, ammonium sulfate, ammonium acetate, and ammonium phosphate, and other nitrogen-containing compounds, peptones, meat extracts, yeast extracts, corn steep liquor, casein hydrolysates, soybean meal and soybean meal hydrolysates, and various fermented bacterial cells and digested products thereof.

Examples of the inorganic salt include potassium dihydrogen phosphate, dipotassium phosphate, magnesium phosphate, magnesium sulfate, sodium chloride, iron(II) sulfate, manganese sulfate, copper sulfate, and calcium carbonate.

In the method for producing CDP-choline by the fermentation method, when the microorganisms used do not have the ability to produce CTP and P-choline that are substrates for the CTP:phosphocholine cytidylyltransferase, CTP and P-choline are added to the culture medium.

In addition, in the method for producing CDP-choline by the fermentation method, when the microorganisms used do not have the ability to produce CTP and P-choline that are substrates of the CTP:phosphocholine cytidylyltransferase, CTP and P-choline may be supplied to the recombinant microorganisms of the present invention by co-culturing a microorganism having the ability to produce CTP and/or P-choline with the recombinant microorganism of the present invention, instead of adding CTP and P-choline to the culture medium.

Culture is generally preferably performed under aerobic conditions such as shaking culture or deep aeration stirring culture. The culture temperature is preferably 15°C to 40°C, and the culture time is generally 5 hours to 7 days. The pH during the culture is preferably maintained at 3.0 to 9.0. The pH is adjusted using an inorganic or organic acid, an alkaline solution, urea, calcium carbonate, ammonia, or the like.

If necessary, antibiotics such as ampicillin and tetracycline may be added to the culture medium during the culture. When a microorganism transformed with an expression vector is cultured using an inducible promoter as a promoter, an inducer may be added to the culture medium as necessary.

For example, isopropyl-β-D-thiogalactopyranoside or the like may be added to the culture medium when a microorganism transformed with an expression vector is cultured using a lac promoter, and indole acrylic acid or the like may be added to the culture medium when a microorganism transformed with an expression vector is cultured using a trp promoter.

CDP-choline can be produced by producing CDP-choline in the culture by means of the above culture. The quantification of CDP-choline can be performed using HPLC (for example, an analyzer SPD-M20A manufactured by Shimadzu Corporation).

The CDP-choline can be collected from the culture by a combination of an ion exchange resin method, a precipitation method, and other known methods. When CDP-choline is accumulated in bacterial cells, CDP-choline can be collected, by using an ion exchange resin method or the like, from a supernatant obtained by, for example, crushing the bacterial cells with ultrasonic waves or the like and removing the bacterial cells by centrifugation.

### (II) Method for Producing CDP-choline Using Culture of Recombinant Microorganism and Treated Product of Culture

A method for producing CDP-choline using a culture of a recombinant microorganism and a treated product of the culture is characterized in that a substrate and a culture of a recombinant microorganism having an ability to produce heterologous CTP:phosphocholine cytidylyltransferase or a treated product of the culture used as an enzyme source are present in an aqueous medium to produce CDP-choline in the aqueous medium. The production method may include, for example, producing CDP-choline in an aqueous medium, then accumulating CDP-choline, and collecting CDP-choline from the aqueous medium.

The method and the culture medium for culturing a microorganism are the same as those described above in (I).

In the present specification, examples of the treated product of the culture include enzyme sources such as a concentrate of the culture, a dried product of the culture, bacterial cells obtained by centrifuging or filtering the culture, a dried product of the bacterial cells, a freeze-dried product of the bacterial cells, a surfactant-treated product of the bacterial cells, a solvent-treated product of the bacterial cells, an enzyme-treated product of the bacterial cells, and immobilized bacterial cells, those containing viable bacterial cells that maintain the same functions as the culture, an ultrasonic-treated product of the bacterial cells, a mechanically pulverized product of the bacterial cells, a crude enzyme extract obtained from the treated bacterial cells, and a purified enzyme obtained from the treated bacterial cells.

Among them, a concentrate of the culture, a dried product of the culture, bacterial cells obtained by centrifuging or filtering the culture, a dried product of the bacterial cells, a freeze-dried product of the bacterial cells, a surfactant-treated product of the bacterial cells, a solvent-treated product of the bacterial cells, an enzyme-treated product of the bacterial cells, and immobilized bacterial cells, those containing viable bacterial cells that maintain the same functions as the culture, an ultrasonic-treated product of the bacterial cells, and a mechanically pulverized product of the bacterial cells are preferably exemplified. A concentrate of the culture, a dried product of the culture, bacterial cells obtained by centrifuging or filtering the culture, a dried product of the bacterial cells, a freeze-dried product of the bacterial cells, a surfactant-treated product of the bacterial cells, a solvent-treated product of the bacterial cells, an enzyme-treated product of the bacterial cells, and immobilized bacterial cells, and those containing viable bacterial cells that maintain the same functions as the culture are most preferably exemplified.

The amount of the CTP:phosphocholine cytidylyltransferase as an enzyme source is preferably 1 g/l to 500 g/l, and more preferably 1 g/l to 300 g/l.

The concentration of the substrate is preferably 0.1 mM to 10 M, and more preferably 1 mM to 1 M. Examples of the substrate include CTP and P-choline.

Examples of the aqueous medium include a buffer solution such as water, a phosphate, a carbonate, an acetate, a borate, a citrate, and Tris, alcohols such as methanol and ethanol, esters such as ethyl acetate, ketones such as acetone, and amides such as acetamide. In addition, a culture solution of a microorganism used as an enzyme source can be used as the aqueous medium.

In a CDP-choline generation reaction, a chelating reagent such as phytic acid, a surfactant, or an organic solvent may be added as necessary. Examples of the surfactant include nonionic surfactants such as polyoxyethylene octadecylamine (for example, NYMEEN S-215, manufactured by NOF Corporation), cationic surfactants such as cetyltrimethylammonium bromide and alkyldimethylbenzylammonium chloride (for example, CATION F2-40E, manufactured by NOF Corporation), anionic surfactants such as lauroyl sarcosionate, and tertiary amines such as alkyldimethylamine (for example, tertiary amine FB, manufactured by NOF Corporation). Any surfactant may be used as long as it promotes the production of CDP-choline. One of the above surfactants may be used, or several kinds thereof may be mixed and used. The surfactant is generally used at a concentration of 0.1 g/l to 50 g/l.

Examples of the organic solvent include xylene, toluene, aliphatic alcohols, acetone, and ethyl acetate, and the organic solvent is generally used at a concentration of 0.1 ml/l to 50 ml/l. The CDP-choline production reaction is performed in an aqueous medium under the conditions of pH 5 to 10, preferably pH 6 to 8, and 20°C to 50°C for 1 hour to 96 hours. In order to promote the production reaction, adenine, adenosine-5'-monophosphate (AMP), adenosine-5'-triphosphate (ATP), magnesium sulfate, magnesium chloride, or the like may be added. Adenine, AMP, and ATP are generally used at concentrations of 0.01 mM to 100 mM.

The CDP-choline generated in the aqueous medium can be quantified and collected by the method described above in (I).

### EXAMPLE

Examples of the present invention are shown below, but the present invention is not limited to these Examples.

### [Analysis Example]

The analysis of CDP-choline and dCDP-choline using the HPLC method was performed using an analyzer SPD-M20A manufactured by Shimadzu Corporation under the following conditions.
Column: Shodex Asahipak
Analysis temperature: 50°C
Flow rate: 0.5 ml/min
Eluent composition: 30 mM potassium dihydrogen phosphate/10% acetonitrile (pH 3.5)
Detector: SPD-M20A

Solutions containing CDP-choline (manufactured by Kyowa Hakko Bio Co., Ltd.) at respective concentrations of 0 g/L, 0.005 g/L, 0.01 g/L, and 0.02 g/L were subjected to analysis as a standard, and a calibration curve was created from an area value of a peak at a retention time of 10.56 min. A sample was diluted to a concentration of 0.005 g/L to 0.02 g/L and subjected to the analysis, and CDP-choline contained in the sample was quantified using the calibration curve from the area value of the peak at the retention time of 10.56 min. Since a standard of dCDP-choline was difficult to obtain, dCDP-choline was quantified using the calibration curve of CDP-choline from an area value of a peak at a retention time of 9.37 min.

### [Example 1] Formation of Microorganism Obtained by Transforming Parent Strain with DNA Encoding CTP:phosphocholine cytidylyltransferase (SplicC) Derived from Streptococcus pneumoniae

PCR was performed using a chromosomal DNA (GenBank: CP053210.1) having the nucleotide sequence represented by SEQ ID NO: 1 as a template and using DNAs consisting of the nucleotide sequences represented by SEQ ID NOs: 2 and 3 as a primer set. A DNA fragment of about 700 bp containing a DNA consisting of the nucleotide sequence represented by SEQ ID NO: 1 was purified.

PCR was performed using pET21a (manufactured by Novagen), which is an expression vector, as a template, and using DNAs consisting of the nucleotide sequences represented by SEQ ID NOs: 4 and 5 as a primer set, and a DNA fragment of about 5.2 kbp (hereinafter referred to as pET21a Inverse fragment) was purified.

The above two types of DNA fragments were ligated using an In-Fusion HD Cloning Kit (manufactured by Takara Bio Inc.), and the ligated reaction solution was used to transform Escherichia coli BL21 strain, thereby forming BL21/pET21a-SplicC (the amino acid sequence of the insert is shown in SEQ ID NO: 6).

### [Example 2] Formation of Microorganism Obtained by Transforming Parent Strain with DNA Encoding CTP:phosphocholine cytidylyltransferase (LslicC) Derived from Lactobacillus salivariuse

PCR was performed using a chromosomal DNA (GenBank: CP011403.1) having the nucleotide sequence represented by SEQ ID NO: 7 as a template and using DNAs consisting of the nucleotide sequences represented by SEQ ID NOs: 8 and 9 as a primer set. A DNA fragment of about 700 bp containing a DNA consisting of the nucleotide sequence represented by SEQ ID NO: 7 was purified.

The DNA fragment and a pET21a Inverse fragment were ligated using an In-Fusion HD Cloning Kit, and the ligated reaction solution was used to transform Escherichia coli BL21 strain, thereby forming BL21/pET21a-LslicC (the amino acid sequence of the insert is shown in SEQ ID NO: 10).

### [Example 3] Formation of Microorganism Obtained by Transforming Parent Strain with DNA Encoding CTP:phosphocholine cytidylyltransferase (AhlicC) Derived from Anaerococcus hydrogenalis

PCR was performed using a chromosomal DNA (GenBank: NZ_ABXA01000041.1) having the nucleotide sequence represented by SEQ ID NO: 11 as a template and using a DNA consisting of the nucleotide sequences represented by SEQ ID NOs: 12 and 13 as a primer set. A DNA fragment of about 700 bp containing a DNA consisting of the nucleotide sequence represented by SEQ ID NO: 11 was purified.

The DNA fragment and a pET21a Inverse fragment were ligated using an In-Fusion HD Cloning Kit, and the ligated reaction solution was used to transform Escherichia coli BL21 strain, thereby forming BL21/pET21a-AhlicC (the amino acid sequence of the insert is shown in SEQ ID NO: 14).

Table 1 shows the results of analyzing the identity of the amino acid sequences encoding SplicC, LslicC, and AhlicC by the Lipman-Pearson method.

**[Table 1]**

| Identity (%) | SplicC | LslicC | AhlicC |
|---|---|---|---|
| SplicC | | 33 | 31 |
| LslicC | 33 | | 62 |
| AhlicC | 31 | 62 | |

As shown in Table 1, LslicC and AhlicC showed an identity of 60% or more. On the other hand, the identities of SplicC with LslicC and AhlicC were as low as about 30% and were less than 60%.

### [Example 4] Production of CDP-choline or dCDP-choline using CTP or dCTP and P-choline as Substrates

BL21/pET21a-SplicC, BL211pET21a-LslicC, and BL21/pET21a-AhlicC were inoculated into a thick test tube containing 5 mL of a LB culture medium [Bacto Tryptone (manufactured by Difco) of 10 g/l, yeast extract (manufactured by Difco) of 5 g/l, sodium chloride of 10 g/l] containing 100 mg/ml of ampicillin, and were cultured at 30°C for 16 hours. The culture solution was inoculated at 1% into a baffle-equipped conical flask containing 50 ml of a LB culture medium containing 100 mg/ml of ampicillin, and cultured at 30°C and 220 rpm for 24 hours. At 2 hours after the start of culture, 0.1 mM of IPTG was added. Fifty milliliters of the culture solution was centrifuged to obtain wet bacterial cells.

Fifty milliliters of each culture solution of each strain was suspended in 5 ml of suspension buffer [50 mM phosphate buffer solution (pH 7.0)], followed by being subjected to ultrasonic crushing. After the crushing, the solution was centrifuged at 5,800×g for 10 minutes to precipitate uncrushed bacterial cells, and then the supernatant was collected. The supernatant was used for a reaction by quantifying the amount of protein by using the Bradford method.

The reaction was performed by adding the supernatant protein solution described above to a reaction solution containing 150 mM of dipotassium hydrogen phosphate-HCl (pH 7.0) (manufactured by Fuji Film Wako Pure Chemical Industries, Ltd.), 25 mM of magnesium chloride hexahydrate (manufactured by Kanto Chemical Industry Co., Ltd.), 5 mM of CTP or dCTP (both manufactured by Sigma-Aldrich Corporation), and 5 mM of P-choline (manufactured by Tokyo Chemical Industry Co., Ltd.) so as to have a final concentration of 0.1 mg/ml, and shaking at 30°C and 1,000 rpm. At a time point at which 10 minutes elapsed from the start of the reaction, 0.6N acetic acid (Fuji Film Wako Pure Chemical Industries, Ltd.) was added in an amount of 1/5 to complete the reaction.

The test was independently performed three times, and CDP-choline and dCDP-choline were quantified by HPLC to determine the average ± standard deviation. The results are shown in Table 2 and Figure.

**[Table 2]**

| | SplicC | LslicC | AhlicC |
|---|---|---|---|
| CDP-choline (mM) | 0.950±0.101 | 1.717±0.026 | 1.666±0.149 |
| dCDP-choline (mM) | 0.749±0.057 | 0.036±0.010 | 0.252±0.067 |
| dCDP-choline/CDP-choline | 0.790±0.035 | 0.021±0.006 | 0.149±0.030 |

As shown in Table 2 and Figure, values of dCDP-choline/CDP-choline were lower in LslicC and AhlicC than in SplicC whose crystal structure was clear, and the reactivity to dCTP was 0.15 or less when the reactivity to CTP was defined as 1. From these results, it was shown that LslicC and AhlicC exhibited excellent substrate specificity in which the reactivity to CTP is higher than the reactivity to dCTP. Further, in consideration of the identity of the amino acid sequences shown in Table 1, it was shown that a polypeptide having an identity of 60% or more with the amino acid sequence of LslicC (SEQ ID NO: 10) or the amino acid sequence of AhlicC (SEQ ID NO: 14) exhibited excellent substrate specificity in which the reactivity to CTP is higher than the reactivity to dCTP.

Although the present invention has been described in detail with reference to specific embodiments, it will be apparent to those skilled in the art that various changes and modifications can be made without departing from the spirit and scope of the present invention. The present application is based on a Japanese Patent Application No. 2021-147985 filed on September 10, 2021, the entire contents of which are incorporated herein by reference. All references cited herein are incorporated in their entirety.

### [Sequence Listing Free Text]

SEQ ID NO: 1: nucleotide sequence of SplicC
SEQ ID NO: 2: nucleotide sequence of SplicC cloning primer_Fw
SEQ ID NO: 3: nucleotide sequence of SplicC cloning primer_Rv
SEQ ID NO: 4: nucleotide sequence of pET21a Inverse primer_Fw
SEQ ID NO: 5: nucleotide sequence of pET21a Inverse primer_Rv
SEQ ID NO: 6: amino acid sequence of insert of pET21a-SplicC
SEQ ID NO: 7: nucleotide sequence of LslicC
SEQ ID NO: 8: nucleotide sequence of LslicC cloning primer_Fw
SEQ ID NO: 9: nucleotide sequence of LslicC cloning primer_Rv
SEQ ID NO: 10: amino acid sequence of insert of pET21a-LslicC
SEQ ID NO: 11: nucleotide sequence of AhlicC
SEQ ID NO: 12: nucleotide sequence of AhlicC cloning primer_Fw
SEQ ID NO: 13: nucleotide sequence of AhlicC cloning primer_Rv
SEQ ID NO: 14: amino acid sequence of insert of pET21a-AhlicC

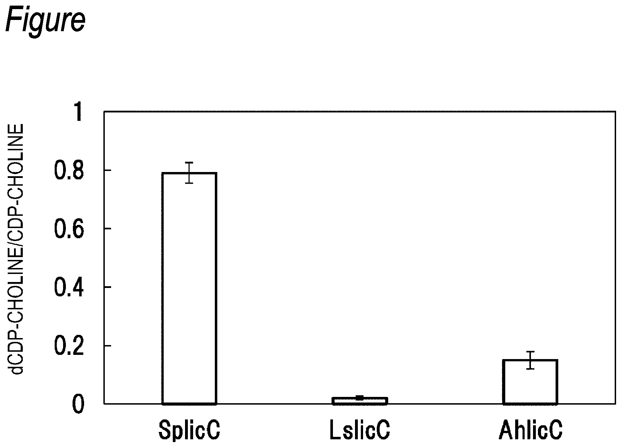

## Claims

1. A recombinant microorganism having an ability to produce heterologous CTP:phosphocholine cytidylyltransferase, wherein the CTP:phosphocholine cytidylyltransferase is any one polypeptide selected from the following (A1) to (A3) and (B1) to (B3):
(A1) a polypeptide consisting of the amino acid sequence represented by SEQ ID NO: 10;
(A2) a polypeptide having a CTP:phosphocholine cytidylyltransferase activity and consisting of an amino acid sequence having an identity of at least 60% or more with the amino acid sequence represented by SEQ ID NO: 10;
(A3) a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 10;
(B1) a polypeptide consisting of the amino acid sequence represented by SEQ ID NO: 14;
(B2) a polypeptide having a CTP:phosphocholine cytidylyltransferase activity and consisting of an amino acid sequence having an identity of at least 60% or more with the amino acid sequence represented by SEQ ID NO: 14; and
(B3) a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 14.

2. The recombinant microorganism according to claim 1, wherein any one DNA selected from the following (a1) to (a3) and (b1) to (b3) is introduced in an expressible manner:
(a1) a DNA encoding the amino acid sequence represented by SEQ ID NO: 10;
(a2) a DNA that encodes a polypeptide having a CTP:phosphocholine cytidylyltransferase activity and consisting of an amino acid sequence having an identity of at least 60% or more with the amino acid sequence represented by SEQ ID NO: 10;
(a3) a DNA that encodes a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 10;
(b1) a DNA encoding the amino acid sequence represented by SEQ ID NO: 14;
(b2) a DNA that encodes a polypeptide having a CTP:phosphocholine cytidylyltransferase activity and consisting of an amino acid sequence in which 1 to 20 amino acids are deleted, substituted, inserted, or added in the amino acid sequence represented by SEQ ID NO: 14; and
(b3) A DNA that consists of a DNA having an identity of at least 60% or more with the DNA encoding the amino acid sequence represented by SEQ ID NO: 14, and encodes a polypeptide having a CTP:phosphocholine cytidylyltransferase activity

3. The recombinant microorganism according to claim 1 or 2, which is Escherichia coli.

4. The recombinant microorganism according to any one of claims 1 to 3, wherein the CTP:phosphocholine cytidylyltransferase has reactivity to dCTP of 0.75 or less when reactivity to CTP is defined as 1.

5. A method for producing CDP-choline, the method comprising producing CDP-choline using the recombinant microorganism according to any one of claims 1 to 4.
